(19) Europäisches Patentamt
European Patent Office
Office européen des brevets

(11) **EP 2 525 782 B1**

(12) # EUROPEAN PATENT SPECIFICATION

(45) Date of publication and mention
of the grant of the patent:
**13.03.2019 Bulletin 2019/11**

(21) Application number: **11732743.7**

(22) Date of filing: **06.01.2011**

(51) Int Cl.:
*A61K 9/14* (2006.01)    *A61K 31/436* (2006.01)
*A61K 47/06* (2006.01)    *A61K 47/14* (2017.01)
*A61K 9/51* (2006.01)

(86) International application number:
**PCT/IN2011/000006**

(87) International publication number:
**WO 2011/086574 (21.07.2011 Gazette 2011/29)**

(54) **FORMULATIONS OF NANO-CARRIERS AND METHODS OF PREPARING THE SAME**

FORMULIERUNGEN AUS NANOTRÄGERN UND VERFAHREN ZU IHRER HERSTELLUNG

PRÉPARATIONS DE NANO-VÉHICULES ET PROCÉDÉS DE PRÉPARATION ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.01.2010 IN 133MU2010**

(43) Date of publication of application:
**28.11.2012 Bulletin 2012/48**

(73) Proprietors:
• **Concept Medical Research Private Limited**
**Surat 395 001 (IN)**
• **Envision Scientific Private Limited**
**Surat 395 002 Gujarat (IN)**

(72) Inventors:
• **DOSHI, Manish**
**Surat 395 017 Gujarat (IN)**
• **SHERDIWALA, Divyesh**
**Surat 395 017 Gujarat (IN)**
• **SOJITRA, Prakash**
**Surat 395 006 Gujarat (IN)**

(74) Representative: **Viering, Jentschura & Partner
mbB**
**Patent- und Rechtsanwälte**
**Am Brauhaus 8**
**01099 Dresden (DE)**

(56) References cited:
**WO-A2-2004/069169      WO-A2-2008/145750**
**WO-A2-2010/137037      WO-A2-2010/140163**
**CN-A- 1 493 361      CN-A- 101 167 699**
**US-A1- 2005 129 727      US-A1- 2009 110 739**

• **BOWEN P: "Particle Size Distribution
Measurement from Millimeters to Nanometers
and from Rods to Platelets", JOURNAL OF
DISPERSION SCIENCE AND TECHNOLOGY,
TAYLOR AND FRANCIS GROUP, NEW YORK, NY,
US, vol. 23, no. 5, 1 January 2002 (2002-01-01),
pages 631-662, XP009102859, ISSN: 0193-2691,
DOI: 10.1081/DIS-120015368**
• **MARCEL DE CUYPER ET AL.: 'Attachment of
Water-Soluble Proteins to the Surface of
(Magnetizable) Phospholipid Colloids via
NeutrAvidin-Derivatized Phospholipids.' J
COLLOID AND INTERFACE SCI. vol. 245, no. 2,
2002, pages 274 - 280, XP008162490**

**Description**

## FIELD OF THE INVENTION

The invention generally relates to formulations of nano-carriers and methods of preparing the formulations of nano-carriers.

## BACKGROUND OF THE INVENTION

With advancements in use of nano-technology in the field of medical science, the nano-sized particles have gained importance in drug delivery systems. Generally, polymers are used along with the nano-sized particles of drugs for delivering these nano-sized particles of drugs to a target site. Nano-particles of a drug encapsulated with a polymer may produce inflammation at the target site. Further, due to improper degradation of the polymer, the drug may be delivered in an uncontrolled manner. Also, semi-degradation of polymers may result in "knife effect" and/or "Edge effect" at the target site. Moreover, when polymers are used for loading a drug on the medical devices, a very low amount of the drug penetrates into the tissues of blood vessels.

Therefore, there is a need in the art for formulations of nano-carriers that may achieve better in-tissue drug diffusion and thereby achieve maximum therapeutic effect with a minimum amount of the drug delivered to a target site. Further, there is a need in the art for methods of encapsulating nano-particles of the drug to form nano-carriers without using polymers.

WO 2004/069169 A2 discloses nanocapsules comprising a therapeutic agent, a polyelectrolyte multilayer shell encapsulating said therapeutic agent, and a tissue specific ligand attached to an outer surface of said polyelectrolyte multilayer shell.

"Bowen P.: Particle Size Distribution Measurement for Millimeters to Nanometers and from Rods to Platelets", Journal of Dispersion Science and Technology, Taylor and Francis Group, New York, NY, US, vol. 23, no.5, 1. January 2002 (2002-01-01'), pages 631-662, XP009102859, ISSN: 0193-2691, DOI:10.1081/DIS-120015368".

According to the present invention, a method for preparing nano-carriers is provided, which method includes the features of claim 1. The dependent claims show some examples of such a method.

## BRIEF DESCRIPTION OF FIGURES

FIG. 1 illustrates the size distribution of nano-particles of Egg phospholipid as detected by Malvern Zeta Sizer (ZS90) in accordance with Example 1.

FIG. 2 illustrates the size distribution of nano-carriers as detected by Malvern Zeta Sizer (ZS90) in accordance with Example 1.

FIG. 3 illustrates the zeta potentials nano-carriers detected by Malvern Zeta Sizer (ZS90) in accordance with Example 1.

FIG 4 illustrates a chromatogram obtained by HPLC for the calculation of sirolimus in solution A4 in accordance with Example 1.

FIG. 5 depicts an optical microscopy image of the coated stent system in accordance with Example 2.

FIG. 6 illustrates a chromatogram for the standard solution in accordance with Example 3.

FIG. 7 illustrates a chromatogram for the sample solution in accordance with Example 3.

## DETAILED DESCRIPTION OF THE INVENTION

Before describing in detail embodiments that are in accordance with the invention, it should be observed that the embodiments reside primarily in formulations of nano-carriers and methods of preparing the formulations of nano-carriers. Accordingly, the formulation components and the method steps have been described to include only those specific details that are pertinent to understanding the embodiments of the invention so as not to obscure the disclosure with details that will be readily apparent to those of ordinary skill in the art having the benefit of the description herein.

In this document, the terms "comprises", "comprising" or any other variation thereof, are intended to cover a non-exclusive inclusion, such that a process, method that comprises a list of elements does not include only those elements but may include other elements not expressly listed or inherent to such process, method. An element preceded by "comprises ...a" does not, without more constraints, preclude the existence of additional identical elements in the process, method that comprises the element.

Further, before describing in detail embodiments that are in accordance with the invention, it should be observed that all the scientific and technical terms used herein for describing the invention have same meanings as would be understood by a person skilled in the art.

Generally speaking, pursuant to various embodiments, the invention discloses a method of preparing nano-carriers. The method includes preparing an organic solution of one or more drugs by dissolving the one or more drugs in one or more organic solvents. The one or more drugs may be selected from one or more of, but are not limited to, an anti-restenotic agent, an anti-proliferative agent, an anti-inflammatory agent, an anti-neoplastic agent, an anti-coagulant agent, an anti-fibrin agent, an antithrombotic agent, an anti-mitotic agent, an antibiotic agent, an anti-allergic agent and an antioxidant, an anti-proliferative agent, estrogens, a protease inhibitor, antibodies, an immunosuppressive agent, a cytostatic agent, a cytotoxic agent, a calcium channel blocker, a phosphodiesterase inhibitor, a prostaglandin inhibitor, a dietary supplement, vitamins, an anti-platelet aggregating agent and genetically engineered epithelial cells.

Examples of a drug include, but are not limited to, sirolimus, tacrolimus, paclitaxel, clobetasol, dexamethasone, genistein, heparin, beta-estadiol, rapamycin, everolimus, ethylrapamycin, zotarolimus, ABT-578, Biolimus A9, docetaxel, methotrexate, azathioprine, vincristine, vinblastine, fluorouracil, doxorubicin hydrochloride, mitomycin, myomycine, novolimus, sodium heparin, a low molecular weight heparin, a heparinoid, hirudin, argatroban, forskolin, vapiprost, prostacyclin, a prostacyclin analogue, dextran, D-phe-pro-arg-chloromethylketone, dipyridamole, glycoprotein IIb/IIIa, recombinant hirudin, bivalirudin, nifedipine, colchicines, lovastatin, nitroprusside, suramin, a serotonin blocker, a steroid, a thioprotease inhibitor, triazolopyrimidine, a nitric oxide or nitric oxide donor, a super oxide dismutase, a super oxide dismutase mimetic, estradiol, aspirin, angiopeptin, captopril, cilazapril, lisinopril, permirolast potassium, alpha-interferon, bioactive RGD and salts, esters or analogues thereof.

These one or more drugs are dissolved in one or more organic solvents. The one or more organic solvents may be, one or more of, but are not limited to, one or more low boiling point organic solvents and one or more organic solvents with low evaporation temperatures. Examples of the one or more organic solvents may include, but are not limited to, acetone, methanol, ethanol, iso-propyl alcohol, 1-butanol, 2-butanol, 2-butanone, acetonitrile, carbon tetrachloride, chlorobenzene, diethyl ether, dimethylether dimethyl-formamide (DMF), dimethyl sulfoxide (DMSO), ethyl acetate, chloromethane, dichloromethane and the like. In an embodiment, the organic solution of the one or more drugs is prepared by dissolving macro-crystals of one of sirolimus (rapamycin) and paclitaxel in one or more of ethyl alcohol and chloromethane.

Subsequent to preparing the organic solution of the one or more drugs, a predetermined amount of water having one or more dissolved surfactants is added to the organic solution of the one or more drugs. The predetermined amount of water may depend upon an amount of the one or more organic solvents used for preparing the organic solution of the one or more drugs. A ratio of the predetermined amount of water to the amount of the one or more organic solvents used for preparing the organic solution of the one or more drugs may range from 1:100 to 100:1. In an embodiment, the ratio of the predetermined amount of water to the amount of the one or more organic solvents used for preparing the organic solution of the one or more drugs is selected based on one or more re-precipitation points associated with the one or more drugs. For example, a ratio of the predetermined amount of water to the amount of the one or more organic solvents ranges from one part of the one or more organic solvents to more than one part of water up to the one or more re-precipitation points.

The one or more dissolved surfactants present in the water may be one or more of, but are not limited to, a surfactant with a Hydrophilic-Lipophilic Balance (HLB) ranging from 3.5 to 16, a cationic emulsifier, an anionic emulsifier, a zwitter ionic emulsifier and a nonionic emulsifier. Examples of the one or more dissolved surfactants may include, but are not limited to, Tween-80, Tween-60, Tween 20, lauryl alcohol ethoxylate and tridecyl alcohol ethoxylate. A ratio of the predetermined amount of water to weight of the one or more surfactants may range from $1*10^{-6}$ to 10 % w/v. In an embodiment, the one or more surfactants are Tween- 80 and the ratio of the predetermined amount of water to the weight of the one or more surfactants is 20:1. The predetermined amount of water having the one or more dissolved surfactants is added to the organic solution of the one or more drugs to obtain a first mixture.

The method further includes preparing an organic solution of one or more biological agents using the one or more organic solvents. The one or more biological agents may be selected from one or more of, but are not limited to, drug carriers, excipients, blood components, excipients derived from blood, naturally occurring phospholipids, solid lipid nano-particles, phospholipids obtained from a living animal, synthetically derived phospholipids, lipoids, vitamins and sugar molecules.

Examples of the one or more biological agents may include, but are not limited to, steroids, vitamins, estradiol, esterified fatty acids, non esterified fatty acids, glucose, inositol, L-lactate, lipoproteins, carbohydrates, tricalcium phosphate, precipitated calcium phosphate, calcium phosphate tribasic, substances derived from at least one of human, egg and soybean, phospholipon 80H, phospholipon 90H, Lipoid S75, Lipoid E80, Intralipid 20, Lipoid EPC, Lipoid E75, lipids obtained from egg, lipids obtained from soya, phosphatidylcholine, phosphatidylglycerol, phosphatidylinositol, phosphatidylserine, phosphatidic acid, cardiolipin, and phosphatidylethanolamine.

The one or more organic solvents may be selected from one or more of, but are not limited to, one or more low boiling point organic solvents and one or more organic solvents with low evaporation temperatures. Examples of the one or more organic solvents may include, but are not limited to, acetone, methanol, ethanol, iso-propyl alcohol, 1-butanol, 2-butanol, 2-butanone, acetonitrile, carbon tetrachloride, chlorobenzene, diethyl ether, dimethylether dimethylformamide (DMF), dimethyl sulfoxide (DMSO), ethyl acetate, chloromethane, dichloromethane and the like. In an embodiment, the organic solution of the one or more biological agents is prepared by dissolving macro-particles of Lipoid E80 in one or more of ethyl alcohol and chloromethane.

Once the organic solution of the one or more biological agents is prepared, a predetermined amount of water having one or more dissolved surfactants is added to the organic solution of the one or more biological agents. The predetermined amount of water may depend upon an amount of the one or more organic solvents used for preparing the organic solution of the one or more biological agents. A ratio of the predetermined amount of water to the amount of the one or more organic solvents used for preparing the organic solution of the one or more biological agents may range from 1:100 to 100:1. In an embodiment, the ratio of the predetermined amount of water to the amount of the one or more organic solvents used for preparing the organic solution of the one or more biological agents is selected based on one or more re-precipitation points associated with the one or more biological agents. For example, the ratio of the predetermined amount of water to the amount of the one or more organic solvents ranges from one part of the one or more organic solvents to more than one part of water up to one or more re-precipitation points associated with one or more biological agents.

The one or more dissolved surfactants present in the water may be one or more of, but are not limited to, a surfactant with a Hydrophilic-Lipophilic Balance (HLB) ranging from 3.5 to 16, a cationic emulsifier, an anionic emulsifier, a zwitter ionic emulsifier and a nonionic emulsifier. Examples of the one or more dissolved surfactants may include, but are not limited to, Tween-80, Tween-60, Tween 20, lauryl alcohol ethoxylate and tridecyl alcohol ethoxylate. A ratio of the predetermined amount of water to weight of the one or more surfactants may range from $1*10^{-6}$ to 10 % w/v. In an embodiment, the ratio of the predetermined amount of water to the weight of the one or more surfactants is 20:1. The predetermined amount of water having the one or more dissolved surfactants is added to the organic solution of the one or more biological agents to obtain a second mixture.

Subsequently, the first mixture and the second mixture are separately subjected to a homogenization process. The homogenization process may include homogenizing the first mixture and the second mixture separately in an ultra sound homogenizer for a predetermined time at a predetermined frequency. Homogenization of the first mixture and second mixture is performed to obtain a solution of nano-crystals of the one or more drugs and a solution of nano-particles of the one or more biological agents, respectively. The predetermined time and the predetermined frequency are determined based on a desired size of the nano-crystals of the one or more drugs and the nano-particles of the one or more biological agents. The predetermined frequency may range from 1 Hz to 30 KHz and the predetermined time may range from 10 minutes to 200 minutes depending upon the desired size of the nano-crystals of the one or more drugs and the nano-particles of the one or more biological agents. Further, one or more of the predetermined frequency and the predetermined time may be varied during the method to obtain desired size of the nano-crystals of the one or more drugs and the nano-particles of the one or more biological agents without departing from the scope of the invention.

Alternatively, the homogenization of the first mixture and the second mixture may be carried out separately using a high speed homogenizer, a high pressure homogenizer and a ball mill. The nano-crystals of the one or more drugs and the nano-particles of the one or more biological agents obtained may have an average diameter of 10 nm to 5000 nm.

Thereafter, the solution of nano-crystals of the one or more drugs and the solution of nano-particles of the one or more biological agents are mixed and subjected to a homogenization process to obtain a solution of nano-carriers. The homogenization process may include homogenizing the solution of nano-crystals of the one or more drugs and the solution of nano-particles of the one or more biological agents together in an ultra sound homogenizer. The homogenization process is performed for a predetermined time at a predetermined frequency to obtain a solution of nano-crystals of the one or more drugs and a solution of nano-particles of the one or more biological agents. The predetermined time and the predetermined frequency are selected based on a desired size of the nano-carriers. The predetermined frequency may range from 1 Hz to 30 KHz and the predetermined time may range from 10 minutes to 200 minutes depending upon the desired size of the nano-carriers. Further, one or more of the predetermined frequency and the predetermined time may be varied during the method to obtain a desired size of the nano-carriers.

During the homogenization of the solution of nano-crystals of the one or more drugs and the solution of nano-particles of the one or more biological agents, the nano-crystals of the one or more drugs are encapsulated with the nano-particles of the one or more biological agents. In other words, as a result of homogenization, the nano-particles of the one or more biological agents come in contact with the nano-crystals of the one or more drugs and cover the nano-crystals of the one or more drugs to form the nano-carriers. The nano-carriers thus formed include the nano-crystals of the one or more drugs encapsulated with the nano-particles of the one or more biological agents. The nano-carriers may have an average diameter of 10 nm to 5000 nm.

Alternatively, the homogenization process may be carried out using a high speed homogenizer, a high pressure homogenizer and a ball mill to obtain the solution of nano-carriers. The solution of the nano-carriers may be used for coating a medical device using coating methods known in the art. The medical device may be a medical device that may be inserted or implanted in the body for delivering a drug to a target site inside the body. Examples of the medical device may include, but are not limited to, a stent, a balloon, a stent mounted on a balloon (a pre-crimped stent), a balloon catheter, a spinal implant, a dental implant, an osteo-implant, a transdermal drug delivery device and any other medical device that may be coated with the nano-carriers for delivering the nano-carriers to a target site.

Examples of the stent may include, but are not limited to, an endovascular stent, a peripheral vascular stent, a urethral stent, a prostatic stent, a stent graft, a permanently implantable stent, and a temporarily implantable stent. The stent may be made up of, one or more of, but not limited to, a metal, an alloy, a biodegradable polymer, and a non-degradable polymer, SS316L, L605 cobalt-chromium alloy, and nickel-titanium alloy, and any stent that may be coated with the nano-carriers.

The balloon may be any balloon made up of an elastomeric material that may be inflated using suitable inflating means in the percutaneous transluminal angioplasty procedures and that may be coated with the nano-carriers for delivering the nano-carriers to a target site. Examples of the balloon may include, but are not limited to, an angioplasty balloon and any other balloon used for interventional cardiovascular procedures.

The method further includes subjecting the solution of the nano-carriers to one or more of an interfacial extraction process and a dialysis process. In an embodiment, an aqueous solution of the nano-carriers is subjected to the dialysis process. The dialysis process may be carried out using methods and techniques known in the art. For example, the aqueous solution of the nano-carriers may be subjected to the dialysis process using dialysis bags and laminar air flow.

Using the dialysis process, the one or more nano-crystals of the one or more drugs that are not encapsulated by the nano-particles of the one or more biological agents may be removed from the solution of the nano-carriers. Further, the nano-particles of the one or more biological agents that are not utilized in the encapsulation may also be removed from the solution of the nano-carriers. The nano-carriers thus obtained have the surface that is substantially devoid of the nano-crystals of the one or more drugs.

The solution of the nano-carriers may also be subjected to interfacial extraction process prior to the dialysis process or after the dialysis process. The solution of the nano-carriers may be extracted using an interfacial solvent extraction process to obtain an extract of the nano-carriers. The interfacial extraction process includes mixing the solution of the nano-carriers in one or more of the one or more organic solvents to obtain a mixture. The mixture is then shaken for a particular time period and subsequently allowed to stand. After the particular time .period, the mixture is separated into an aqueous phase and an organic phase. The organic phase contains the nano-carriers. The organic phase may then be filtered to remove any impurities to obtain the extract of nano-carriers.

The extract of the nano-carriers may then be coated on the medical device to obtain a medical device coated with the nano-carriers for delivering the nano-carriers to a target site. Alternatively, the extract of the nano-carriers may be subjected to solidification to obtain solidified nano-carriers. The solidified nano-carriers may be used for formulating one of a solid dosage form, a liquid dosage form and a semiliquid dosage form. The solidified nano-carriers may also be used for localized delivery of a drug to a target site. For example, the solidified nano-carriers may be used for localized delivery of the drugs to a tumor. Further, the solidified nano-carriers may be used to formulate injectables. The injectables may be used for localized delivery of the drug in patients with cancer.

Generally speaking, pursuant to various embodiments, the invention also relates to formulations of nano-carriers. The formulations of nano-carriers may include, but are not limited to, the solution of nano-carriers, the nano-carriers, the solidified nano-carriers, a solid dosage form containing the nano-carriers, a liquid dosage form containing the nano-carriers, a semi-solid dosage form containing the nano-carriers.

The nano-carriers include the nano-crystals of the one or more drugs surrounded by the nano-particles of the one or more biological agents. The one or more biological agents are selected such that the one or more biological agents exhibit an affinity for the tissues of a target site. Such an affinity facilitates efficient transferring of the nano-carriers to the target site over a time. Further, owing to the dialysis process, the nano-carriers are substantially devoid of drug crystals on the surface of the nano-carriers. Thus, a direct contact of the one or more drugs with the surface of the medical device may be avoided. Further, the one or more drugs come in contact with the tissues of the target site only when the nano-carrier penetrates into the target site and the one or more biological agents are dissolved. Thus, direct exposure of the one or more drugs to the tissues of the target site and the surface of the medical device is prevented. In addition, as no polymer is used for preparing the nano-carriers, the nano-carriers are less toxic as compared to the formulations or drug delivery medical devices containing polymers. Owing to the properties of the one or more biological agents and the size of the nano-carriers, an enhanced bioavailability of the drugs, an enhanced biocompatibility and an enhanced delivery of a drug to maximum portion of the target site with optimum drug loading may be achieved.

Example 1:

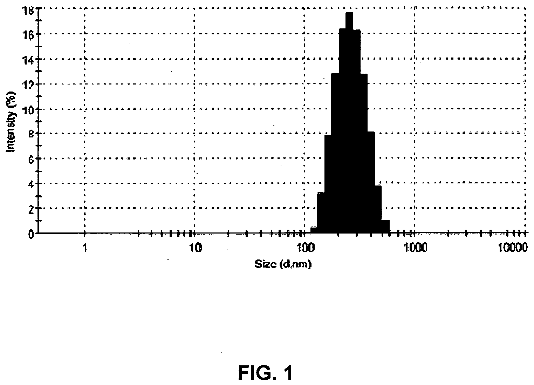

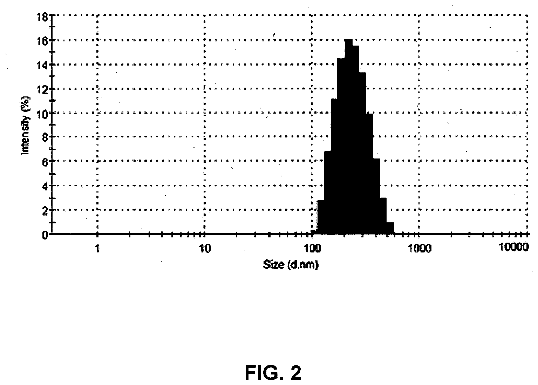

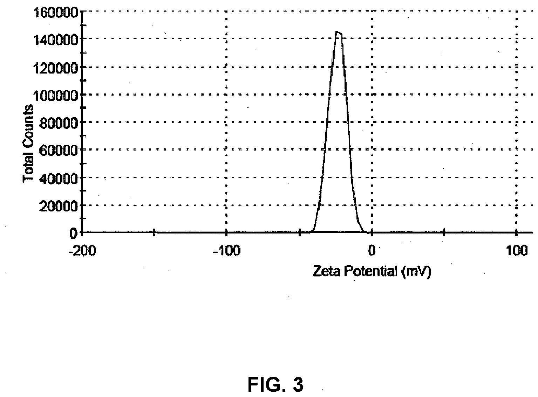

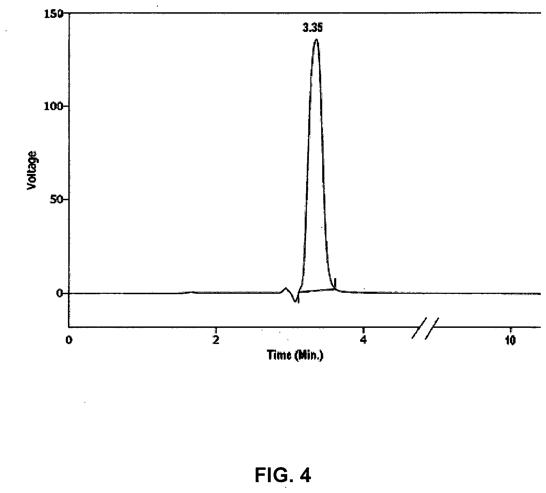

Egg phospholipid was obtained from Lipoid GMBH, Batch No.: 1032313-16/903. Sirolimus was obtained from Fujan Chemicals, China with purity greater than 99.5 %. The water, other solvents and reagents used were of HPLC grade.
Egg phospholipid (200 mg w/w) was dissolved in methanol. 100 ml HPLC grade water and Tween 80 (5 mg) was added to obtain an aqueous solution of Egg phospholipid. The aqueous solution of Egg phospholipid (10 ml) was subjected to ultrasonic homogenization for 20 to 25 minutes in ice-cold water bath to obtain Solution A1. The Solution A1 thus obtained contained nano-particles of Egg phospholipid. The solution A1 was subsequently analyzed for particle size detection using Malvern Zeta Sizer (ZS90) [Malvern, UK] size detector. FIG. 1 illustrates the size distribution of nano-particles of Egg phospholipid as detected by Malvern Zeta Sizer (ZS90). Z-average diameter of the nano-particles of the Egg phospholipid was found to be 246.7 nm with largest diameter up to 531.2 nm.
Sirolimus (10 mg w/w) was dissolved in 5 ml methanol, and then 100 ml of HPLC grade water was added to obtain an aqueous solution of sirolimus by recrystalization. The aqueous solution of sirolimus (100 ml) was subjected to ultrasonic homogenization for 100 to 200 minutes in ice-cold water bath to obtain Solution A2. The Solution A2 thus obtained contained nano-crystals of sirolimus.
After 100 to 180 minutes, 5 ml Solution A1 was added to Solution A2 drop by drop using 5 ml pipette with ultrasonic homogenization process. The resultant mixture was subjected to an ultrasonic homogenization process for another 15 minutes after complete addition to obtain Solution A3. Solution A3 was kept in ultrasonic cleaner water bath (PCI) for 20 minutes. Solution A3 thus obtained contained nano-carriers (nano-crystals of sirolimus surrounded by nano-particles of egg phospholipid). Solution A3 was subsequently analyzed for particle size detection using Malvern Zeta Sizer (ZS90) [Malvern, UK] size detector. FIG. 2 illustrates the size distribution of nano-carriers as detected by Malvern Zeta Sizer (ZS90). The z-average diameters of nano-carriers were found to be 229.7 nm with maximum diameter being up to 615.1 nm.
Zeta potential of nano-carriers was measured to estimate stability of particles. FIG. 3 illustrates the zeta potentials nano-carriers detected by Malvern Zeta Sizer (ZS90). The measured zeta potential of nano-carriers was -24 to -31 mV. It indicates moderate stability of the nano-carriers in an aqueous form.
Solution A3 (Aqueous solution of nano-carriers) was further subjected to extraction with dichloromethane. Solution A3 (10 ml) was transferred to 100 ml separating funnel. 30 ml of dichloromethane was added to the 100 ml separating funnel. The resultant mixture was shaken for 15 minutes and then allowed to stand. Thereafter, two layers i.e. aqueous layer and the dichloromethane layer were observed in the 100 ml separating funnel. The dichloromethane layer was separated from the aqueous layer. Similarly, 20 ml of dichloromethane was added to the 100 ml in the same aqueous solution containing separating funnel to allow extraction. The resultant mixture was shaken for 15 minutes and then allowed to stand. Thereafter, two layers i.e., aqueous layer and the dichloromethane layer were observed in the 100 ml separating funnel. The dichloromethane layer was separated from the aqueous layer. The resultant mixture was shaken for 15 minutes and then allowed to stand. Dichloromethane layer 50 ml was separated and the resultant solution A4 i.e., extracted solution of the nano-carriers was stored in an amber colored small measuring flask with a batch number.
Four batches of the extracted solution of the nano-carriers were mixed in a glass beaker. The glass beaker was kept in a water bath to allow solvent evaporation to obtain about 0.5 mg/ml sirolimus concentration. Further, the solution of nano-carriers was tested for the concentration of sirolimus using HPLC. FIG 4 illustrates a chromatogram obtained by HPLC for the calculation of sirolimus in solution A4.
Area of peak for the solution A4 was calculated. The retention time for the sample solution was found to be 3.35 minutes and the "Sample Area" corresponding to the peak for the sample solution was found to be 1751.241 mV*Sec. Subsequently, the amount of sirolimus present in solution A4 was calculated using the following formula:

$$\text{Amount of Drug (Sirolimus)} = (\text{Sample Area} / \text{Standard Area}) * (\text{Standard Concentration} / \text{Sample Concentration})$$

Therefore,

$$\text{Amount of Drug} = (1715.241/ 1546.577)* (50 / (1/10)) = 554.52 \ \mu g/ml$$

Thus, the concentration of sirolimus in solution A4 was found to be 554.52 µg/ml.

Example 2

Preparation of a medical device coated with nano-carriers:

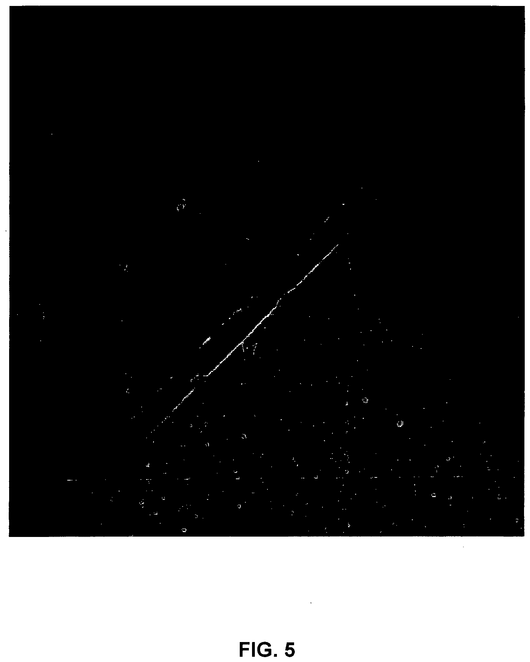

The solution of the nano-carriers i.e. Solution A4 (1.4 ml) was fed into a reservoir of a coating machine. The stent system (Amazonia Croco: 2.75*08 mm) was mounted on a rotating mandrel of the coating machine. The stent system was exposed to an atomization nozzle of the coating machine. The stent system was rotated at about 5 to 40 rpm by rotating the mandrel. Simultaneously, the solution of nano-carriers was sprayed over the stent system at 0.5-4.0 psi inert gas pressure and two oscillations. Thus, the stent system coated with the nano-carriers (hereinafter "the coated stent system") was obtained. The coated stent system was then removed and checked under high resolution microscope for the coating surface smoothness and any foreign particles. FIG. 5 depicts optical microscopy image of the coated stent system. The Labomed Microscope was equipped with a Motic Digital Camera for image capturing.

Example 3

Detection of drug content of the coated stent system:

The amount of sirolimus loaded on the coated stent system was calculated using High Performance Liquid Chromatography (HPLC) analysis. The HPLC operating parameters were selected as: Flow Rate was set at 1.0 ml/min. ($\pm$ 0.01), λ Maxima was set at 277 nm ($\pm$ 1 nm), Column Temperature was set at 50 °C ($\pm$ 2 °C), Sensitivity of detector was set at 0.02 AUFS, injection volume was 20 μL and analysis time was set up to 20 minutes.

HPLC System [Analytical 2010 low pressure gradient equipped with auto sampler (S 5200), UV-Visible Detector (UV 2230), HPLC pump (P2230) and A 2000 Chromatography work station] was used for the HPLC analysis. Column - $C_{18}$ [$RP_{18}$ Length 4.6 mm X 250 mm, particle size 5 μm] was attached with column oven [PCI] for heating. The samples were filtered through the millipore PTFE 0.45-micron syringe filter before analysis to avoid any particulate matters. Pre-calibrated class A grade volumetric flasks were used. Amber coloured glassware was used to protect against light. All the Renchem solvents and reagents used were of HPLC grade. Sirolimus was used as received from Fujan Chemicals, China with purity greater than 99.5%.

Mobile phase included Acetonitrile: Methanol: Water in a concentration ratio of 22:67:11. Mobile phase was subsequently degassed in an ultrasonic cleaner for 10 minutes.

Sirolimus (0.5 mg) was taken in a clean and dry 10 ml Standard Measuring Flask (SMF). The SMF was then filled up to mark with the mobile phase and shaken for 5 to 10 minutes. The SMF was then kept in an ultrasonic cleaner and degassed for 10 minutes. The solution was then filtered through 0.45 micron syringe filter to obtain a standard solution with "Standard Concentration" of 50 μg/ml.

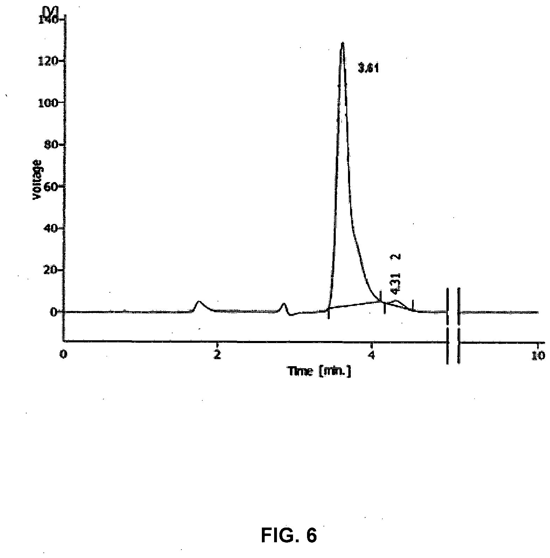

Using the sample vial 20 μL of the standard solution was injected in the HPLC system using auto sampler and a chromatogram for the standard solution was obtained. FIG. 6 illustrates a chromatogram for the standard solution. Subsequently, the area of the peak for the standard solution ("Standard Area") was calculated. The retention time for the standard solution was found to be 3.61 minutes and the "Standard Area" corresponding to the peak for the standard solution was found to be 1546.577 mV*Sec..

For the quantification of the drug content loaded on the stent system, the sample solution was prepared by inserting the coated sent system in 10 ml SMF filled with methanol (10 ml). The SMF was then kept in ultrasonic bath for 10 minutes to allow the sirolimus present in the coated stent system to completely dissolve in the methanol. Thus, the sample solution was obtained.

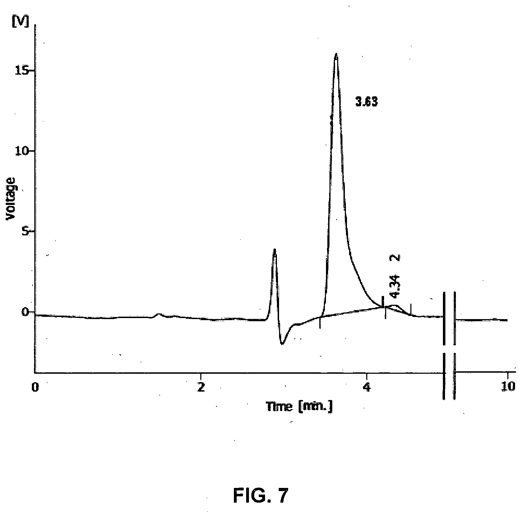

The sample solution was injected in the HPLC injector and a chromatogram for the sample solution was obtained. FIG. 7 illustrates a chromatogram for the sample solution. Subsequently, the area of the peak for the sample solution ("Sample Area") was calculated. The retention time for the sample solution was found to be 3.63 minutes and the "Sample Area" corresponding to the peak for the sample solution was found to be 197.032 mV*Sec. Subsequently, the amount of sirolimus present in the coated stent system was calculated using the following formula:

$$\text{Amount of Drug (loaded on the coated stent system)} = (\text{Sample Area} / \text{Standard Area}) * (\text{Standard Concentration} / \text{Sample Concentration})$$

Therefore,

$$\text{Amount of Drug} = (197.032/1546.577)* (50 / (1/10)) = 63.69 \ \mu g$$

Thus, the Amount of Drug loaded on the 2.75*08 mm coated stent system was found to be 63.69 $\mu g$.

Example 4

Preparation of solidified nano-carriers and/or solid dosage forms of nano-carriers:

Solution 4 was evaporated till dryness. The solid residues were collected and further dried in vacuum at 40 °C to remove residual solvents. The resultant solidified nano-carriers were stored in glass vial in refrigerator at 4-10 °C.

Example 5

Preparation of injectables:

10 mg of the solidified nano-carriers (obtained as explained in Example 4) were dissolved in to the 100 ml ethanol to obtain 0.1 mg/ml solution of the solidified nano-carriers. 1 ml of solution of nano-carriers was filled in to an air tight vial under sterile conditions. Finally, the air tight vial was sealed with purging nitrogen gas to avoid any air contamination. The vials thus obtained were assigned a batch number and stored. Similarly, vials with 1 mg/ml and 5 mg/ml concentration of solidified nano-carriers were prepared and stored.

Example 6

Dialysis treatment of the solution of nano-carriers:

6 mm dialysis bag (Spectra/Por 10 mm flat diameter) was rinsed with hot water. One end of the dialysis bag was tied and then inner side of dialysis bag was rinsed with PBS. The solution A3 (obtained in Example 1) was transferred to the dialysis bag under a laminar flow hood (Alliance Enterprise, INDIA). Open end of the dialysis bag was then clamped with a dialysis tube closure to avoid liquid-air interface contamination. The dialysis bag was immersed in pre-cooled (4 to 10 °C) 500 ml to 1 L of degassed 25 mM Tris-0.15 M NaCl at pH 7.5 (RT) for 12-18 hours and degassed 12 to 18 hours in PBS with pH 7.4 pH. After 24 to 36 hours the dialysis bag containing solution A3 was removed from the media under laminar air flow. The bag was cut with sterile blade above clip carefully and sample was transferred in the close air tight container.

Various embodiments of the invention provide formulations of nano-carriers and methods of preparing the nano-carriers. The formulations of nano-carriers exhibit an enhanced affinity for tissues of a target site thereby facilitating efficient transfer of the nano-carriers to the target site over a time. The invention also provides polymer-free nano-carriers that are less toxic as compared to the formulations or drug delivery medical devices containing polymers. Further, owing to the properties of the one or more biological agents and the size of the nano-carriers, an enhanced bioavailability of the drugs, an enhanced biocompatibility and an enhanced delivery of a drug to maximum portion of the target site with optimum drug loading may be achieved using the formulations of the nano-carriers disclosed herein.

Those skilled in the art will realize that the above-recognized advantages and other advantages described herein are merely exemplary and are not meant to be a complete rendering of all of the advantages of the various embodiments of the invention.

In the foregoing specification, specific embodiments of the present invention have been described. However, one of ordinary skill in the art appreciates that various modifications and changes can be made without departing from the scope of the present invention as set forth in the claims below. Accordingly, the specification and figures are to be regarded in an illustrative rather than a restrictive sense, and all such modifications are intended to be included within the scope of the present invention. The benefits, advantages, solutions to problems, and any element(s) that may cause any benefit, advantage, or solution to occur or become more pronounced are not to be construed as a critical, required, or essential features or elements of any or all the claims. The present invention is defined solely by the appended claims including any amendments made during the pendency of this application and all equivalents of those claims as issued.

**Claims**

**1.** A method of preparing nano-carriers, the method comprising:

mixing an organic solution of a drug and an organic solution of a biological agent separately with a predetermined amount of water having at least one dissolved surfactant to obtain a first mixture and a second mixture respectively;

homogenizing the first mixture and the second mixture separately to obtain a solution of nano-crystals of the drug and a solution of nano-particles of the biological agent respectively;

subjecting the solution of nano-crystals of the drug and the solution of nano-particles of the biological agent together to an ultra-sound homogenization to obtain a solution of nano-carriers; and

performing at least one of an interfacial extraction and a dialysis on the solution of nano-carriers to obtain the nano-carriers.

2. The method of claim 1, wherein interfacial extraction of the solution of nano-carriers yields an extract of the nano-carriers, the extract of the nano-carriers capable of being coated on a medical device.

3. The method of claim 1 further comprising solidifying the nano-carriers to obtain solidified nano-carriers, the solidified nano-carriers capable of being used for at least one of an oral delivery and systemic delivery of the drug.

4. The method of claim 1, wherein the organic solution of the drug comprises the drug and an organic solvent, the organic solvent being selected from at least one of acetone, methanol, ethanol, iso-propyl alcohol, 1-butanol, 2-butanol, 2- butanone, acetonitrile, carbon tetrachloride, chlorobenzene, diethyl ether, dimethylether dimethyl-formamide (DMF), dimethyl sulfoxide (DMSO), ethyl acetate, chloromethane, and dichloromethane.

5. The method of claim 1, wherein the organic solution of the biological agent comprises the biological agent and an organic solvent, the organic solvent being selected from at least one of acetone, methanol, ethanol, iso-propyl alcohol, 1- butanol, 2-butanol, 2-butanone, acetonitrile, carbon tetrachloride, chlorobenzene, diethyl ether, dimethylether dimethyl-formamide (DMF), dimethyl sulfoxide (DMSO), ethyl acetate, chloromethane, and dichloromethane.

6. The method of claim 1, wherein at least one of a ratio of the predetermined amount of water to an amount of the organic solution of the drug and a ratio of the predetermined amount of water to the organic solution of the biological agent ranges from 1 : 100 to 100:1.

7. The method of claim 1, wherein the at least one dissolved surfactant is selected from at least one of a surfactant with a Hydrophilic-Lipophilic Balance (HLB) ranging from 3.5 to 16, Tween-80, Tween-60, Tween 20, lauryl alcohol ethoxylate, tridecyl alcohol ethoxylate, a cationic emulsifier, an anionic emulsifier, a zwitter ionic emulsifier and a nonionic emulsifier.

8. The method of claim 1 , wherein the first mixture and the second mixture are separately homogenized using an ultra-sound homogenizer for a time ranging from 10 minutes to' 200 minutes at a frequency ranging from 1 Hz to 30 kHz in an ice-cold water bath.

9. The method of .claim 1, wherein the solution of nano-crystals of the drug and the solution of nano-particles of the biological agent are together subjected to the ultra-sound homogenization for a time ranging from 10 minutes to 200 minutes at a frequency ranging from 1 Hz to 30 kHz in a ice-cold water bath.

10. The method of claim 1 , wherein the drug is selected from at least one of an anti-restenotic agent, an anti-proliferative agent, an anti-inflammatory agent, an antineoplastic agent, an anti-coagulant agent, an anti-fibrin agent, an antithrombotic agent, an anti-mitotic agent, an antibiotic agent, an anti-allergic agent and an antioxidant, an antiproliferative agent, estrogens, a protease inhibitor, antibodies, an immunosuppressive agent, a cytostatic agent, a cytotoxic agent, a calcium channel blocker, a phosphodiesterase inhibitor, a prostaglandin inhibitor, a dietary supplement, vitamins, anti-platelet aggregating agent and genetically engineered epithelial cells.

11. The method of claim 1, wherein the drug is selected from at least one of sirolimus, tacrolimus, paclitaxel, clobetasol, dexamethasone, genistein, heparin, beta-estadiol, rapamycin, everolimus, ethylrapamycin, zotarolimus, ABT-578, Biolimus A9, docetaxel, methotrexate, azathioprine, vincristine, vinblastine, fluorouracil, doxorubicin hydrochloride, mitomycin, myomycine, novolimus, sodium heparin, a low molecular weight heparin, a heparinoid, hirudin, argatroban, forskolin, vapiprost, prostacyclin, a prostacyclin analogue, dextran, D- phe-pro-arg-chloromethylketone, dipyridamole, glycoprotein IIb/IIIa, recombinant hirudin, bivalirudin, nifedipine, colchicines, lovastatin, nitroprusside, suramin, a serotonin blocker, a steroid, a thioprotease inhibitor, triazolopyrimidine, a nitric oxide or nitric oxide donor, a super oxide dismutase, a super oxide dismutase mimetic, estradiol, aspirin, angiopeptin, captopril, cilazapril, lisinopril, permirolast potassium, alpha-interferon, bioactive RGD and salts, esters or analogues thereof.

12. The method of claim 1, wherein the drug is at least one of sirolimus, tacrolimus, paclitaxel and salts, esters or analogues thereof.

13. The method of claim 1, wherein the biological agent is selected from at least one of a drug carrier, an excipient, a blood component, an excipient derived from blood, a phospholipid, solid lipid nano-particles, a lipoid, a vitamin and a sugar molecule.

14. The method of claim 1, wherein the biological agent is selected from at least one of a steroid, a vitamin, an estradiol, an esterified fatty acid, a non-esterified fatty acid, glucose, inositol, L-lactate, a lipoprotein, a carbohydrate, tricalcium phosphate, precipitated calcium phosphate, a substance derived from at least one of human, egg and soybean, phospholipon 80H, phospholipon 90H, Lipoids S75, Lipoids E80, Intralipid 20, Lipoid EPC, Lipoids E75, a lipid obtained from egg, a lipid obtained from soya, phosphatidylcholine, phosphatidylglycerol, phosphatidylinositol, phosphatidylserine, phosphatidic acid, cardiolipin, and phosphatidylethanolamine.

15. The method of claim 1, wherein the biological agent is selected from at least one of a Lipoid E80 and a phospholipid obtained from egg.

16. The method of claim 1, wherein the nano-carriers comprise the drug encapsulated within the biological agent, wherein the surface of the nano-carriers is devoid of the drug and the nano-carriers are devoid of any polymer.

17. The method of claim 1, wherein size of the nano-carriers ranges from 10 nm to 5000 nm.

**Patentansprüche**

1. Ein Verfahren zum Herstellen von Nano-Trägern, wobei das Verfahren aufweist:

separates Mischen einer organischen Lösung eines Medikaments und einer organischen Lösung eines biologischen Wirkstoffs mit einer vorbestimmten Menge Wasser, welches zumindest ein gelöstes Tensid hat, um ein erstes Gemisch beziehungsweise ein zweites Gemisch zu erhalten;
separates Homogenisieren des ersten Gemisches und des zweiten Gemisches, um eine Lösung von Nano-Kristallen des Medikaments beziehungsweise eine Lösung von Nano-Partikeln des biologischen Wirkstoffs zu erhalten;
gemeinsames Aussetzen der Lösung von Nano-Kristallen des Medikaments und der Lösung von Nano-Partikeln des biologischen Wirkstoffs einer Ultraschall-Homogenisierung, um eine Lösung von Nano-Trägern zu erhalten; und
Anwenden von zumindest einem von einer Grenzflächenextraktion und einer Dialyse an der Lösung von Nano-Trägern, um Nano-Träger zu erhalten.

2. Das Verfahren gemäß Anspruch 1, wobei die Grenzflächenextraktion der Lösung von Nano-Trägern ein Extrakt der Nano-Träger erzeugt, wobei das Extrakt der Nano-Träger in der Lage ist, auf eine medizinische Vorrichtung aufgebracht zu werden.

3. Das Verfahren gemäß Anspruch 1, ferner aufweisend ein Härten der Nano-Träger, um gehärtete Nano-Träger zu erhalten, wobei die gehärteten Nano-Träger in der Lage sind, um für mindestens eines von einer oralen Verabreichung und einer systemischen Verabreichung des Medikaments verwendet zu werden.

4. Das Verfahren gemäß Anspruch 1, wobei die organische Lösung des Medikaments das Medikament und ein organisches Lösungsmittel aufweist, wobei das organische Lösungsmittel ausgewählt ist aus mindestens einem von Aceton, Methanol, Ethanol, Isopropylalkohol, 1-Butanol, 2-Butanol, 2-Butanon, Acetonitril, Kohlenstofftetrachlorid, Chlorobenzen, Diethylether, Dimethylether Dimethylformamid (DMF), Dimethylsulfoxid (DMSO), Ethylacetat, Chloromethan und Dichloromethan.

5. Das Verfahren gemäß Anspruch 1, wobei die organische Lösung des biologischen Wirkstoffs den biologischen Wirkstoff und ein organisches Lösungsmittel aufweist, wobei das organische Lösungsmittel ausgewählt ist aus mindestens einem von Aceton, Methanol, Ethanol, Isopropylalkohol, 1-Butanol, 2-Butanol, 2-Butanon, Acetonitril, Kohlenstofftetrachlorid, Chlorobenzen, Diethylether, Dimethylether Dimethylformamid (DMF), Dimethylsulfoxid (DMSO), Ethylacetat, Chloromethan und Dichloromethan.

6. Das Verfahren gemäß Anspruch 1, wobei mindestens eines von einem Verhältnis der vorbestimmten Menge Wasser zu einer Menge der organischen Lösung des Medikaments und einem Verhältnis der vorbestimmten Menge Wasser zu der organischen Lösung des biologischen Wirkstoffs in einem Bereich von 1:100 bis 100:1 liegt.

7. Das Verfahren gemäß Anspruch 1, wobei das mindestens eine gelöste Tensid ausgewählt ist aus mindestens einem von einem Tensid mit einer Hydrophil-Lipophil-Balance (HLB) in einem Bereich von 3,5 bis 16, Tween-80, Tween-60, Tween-20, Laurylalkoholethoxylat, Tridecylalkoholethoxylat, einem kationischen Emulgator, einem anionischen Emulgator, einem zwitterionischen Emulgator und einem nichtionischen Emulgator.

8. Das Verfahren gemäß Anspruch 1, wobei das erste Gemisch und das zweite Gemisch unter Verwendung eine Ultraschall-Homogenisators für eine Zeit in einem Bereich von 10 Minuten bis 200 Minuten bei einer Frequenz in einem Bereich von 1 Hz bis 30 kHz in einem eiskalten Wasserbad separat homogensiert werden.

9. Das Verfahren gemäß Anspruch 1, wobei die Lösung von Nano-Kristallen des Medikaments und die Lösung von Nano-Partikeln des biologischen Wirkstoffs für eine Zeit in einem Bereich von 10 Minuten bis 200 Minuten bei einer Frequenz in einem Bereich von 1 Hz bis 30 kHz in einem eiskalten Wasserbad gemeinsam der Ultraschall-Homogensierung ausgesetzt werden.

10. Das Verfahren gemäß Anspruch 1, wobei das Medikament ausgewählt ist aus mindestens einem von einem Anti-Restenosewirkstoff, einem anti-proliferativen Wirkstoff, einem entzündungshemmenden Wirkstoff, einem antineoplastischen Wirkstoff, einem Antikoagulanzwirkstoff, einem Anti-Fibrin-Wirkstoff, einem antithrombotischen Wirkstoff, einem anti-mitotischen Wirkstoff, einem antibiotischen Wirkstoff, einem antiallergischen Wirkstoff und einem Antioxidans, einem anti-proliferativen Wirkstoff, Östrogenen, einem Protease-Inhibitor, Antikörpern, einem immunsuppressiven Wirkstoff, einem zytostatischem Wirkstoff, einem zytotoxischen Mittel, einem Kalziumkanalblocker, einem Phosphodiesterase-Inhibitor, einem Prostaglandin-Inhibitor, einem Nahrungsergänzungsmittel, Vitaminen, einem Anti-Plättchenaggregationswirkstoff und gentechnisch veränderten Epithelzellen.

11. Das Verfahren gemäß Anspruch 1, wobei das Medikament ausgewählt ist aus mindestens einem von Sirolimus, Tacrolimus, Paclitaxel, Clobetasol, Dexamethason, Genistein, Heparin, Beta-Estadiol, Rapamycin, Everolimus, Ethylrapamycin, Zotarolimus, ABT-578, Biolimus A9, Docetaxel, Methotrexat, Azathioprin, Vincristin, Vinblastin, Fluorouracil, Doxorubicinhydrochlorid, Mitomycin, Myomycin, Novolimus, Natriumheparin, einem niedermolekularen Heparin, einem Heparinoid, Hirudin, Argatroban, Forskolin, Vapiprost, Prostacyclin, einem Prostacyclin-Analogon, Dextran, D-phe-pro-arg-chlormethylketon, Dipyridamol, Glykoprotein IIb/IIIa, rekombinantem Hirudin, Bivalirudin, Nifedipin, Colchicinen, Lovastatin, Nitroprussid, Suramin, einem Serotoninblocker, einem Steroid, einem Thioprotease-Inhibitor, Triazolopyrimidin, einem Stickoxid oder Stickoxidspender, einer Superoxid-Dismutase, einem Superoxid-Dismutase-Mimetikum, Estradiol, Aspirin, Angiopeptin, Captopril, Cilazapril, Lisinopril, Permirolast-Kalium, Alpha-Interferon, bioaktiven RGD und Salzen, Estern oder Analoga davon.

12. Das Verfahren gemäß Anspruch 1, wobei das Medikament mindestens eines ist von Sirolimus, Tacrolimus, Paclitaxel und Salzen, Externs und Analoga davon.

13. Das Verfahren gemäß Anspruch 1, wobei der biologische Wirkstoff ausgewählt ist aus mindestens einem von einem Medikamententräger, einem Hilfsstoff, einem Blutbestandteil, einen aus Blut abgeleiteten Hilfsstoff, einem Phospholipid, festen Lipid-Nanopartikeln, einem Lipoid, einem Vitamin und einem Zuckermolekül.

14. Das Verfahren gemäß Anspruch 1, wobei der biologische Wirkstoff ausgewählt ist aus zumindest einem von einem Steroid, einem Vitamin, einem Estradiol, einer veresterten Fettsäure, einer nicht-veresterten Fettsäure, Glukose, Inositol, L-Laktat, einem Lipoprotein, einem Kohlehydrat, Tricalciumphosphat, gefälltem Calciumphosphat, einer Substanz, die von mindestens einem von einem Menschen, aus Ei und aus Sojabohne stammt, Phospholipon 80H, Phospholipon 90H, Lipoide S75, Lipoide E80, Intralipid 20, Lipoid EPC, Lipoide E75, einem Lipid aus Ei, einem Lipid aus Soja, Phosphatidylcholin, Phosphatidylglycerin, Phosphatidylinositol, Phosphatidylserin, Phosphatidsäure, Cardiolipin und Phosphatidylethanolamin.

15. Das Verfahren gemäß Anspruch 1, wobei der biologische Wirkstoff ausgwählt ist aus zumindest einem von einem Lipoid E80 und einem aus Ei gewonnenen Phospholipid.

**16.** Das Verfahren gemäß Anspruch 1, wobei die Nano-Träger das in dem biologischen Wirkstoff eingebettete Medikament aufweisen, wobei die Oberfläche der Nano-Träger frei von dem Medikament ist und die Nano-Träger frei von irgendeinem Polymer sind.

**17.** Das Verfahren gemäß Anspruch 1, wobei die Größe der Nano-Träger in einem Bereich von 10 nm bis 5000 nm liegt.

**Revendications**

**1.** Procédé de préparation de nano-véhicules, le procédé comprenant :

le mélange d'une solution organique d'un médicament et d'une solution organique d'un agent biologique séparément avec une quantité prédéterminée d'eau ayant au moins un tensioactif dissous pour que soient respectivement obtenus un premier mélange et un deuxième mélange ;
l'homogénéisation du premier mélange et du deuxième mélange séparément pour que soient respectivement obtenues une solution de nano-cristaux du médicament et une solution de nano-particules de l'agent biologique ;
la soumission de la solution de nano-cristaux du médicament et de la solution de nano-particules de l'agent biologique ensemble à une homogénéisation aux ultrasons pour que soit obtenue une solution de nano-véhicules ; et
la mise en oeuvre d'au moins l'une parmi une extraction à l'interface et une dialyse sur la solution de nano-véhicules pour que soient obtenus les nano-véhicules.

**2.** Procédé selon la revendication 1, dans lequel l'extraction à l'interface de la solution de nano-véhicules engendre un extrait des nano-véhicules, l'extrait des nano-véhicules pouvant être déposé sous forme de revêtement sur un dispositif médical.

**3.** Procédé selon la revendication 1, comprenant en outre la solidification des nano-véhicules pour que soient obtenus des nano-véhicules solidifiés utilisables pour au moins l'une parmi une délivrance par voie orale et une délivrance par voie systémique du médicament.

**4.** Procédé selon la revendication 1, dans lequel la solution organique du médicament comprend le médicament et un solvant organique, le solvant organique étant au moins l'un choisi parmi l'acétone, le méthanol, l'éthanol, l'alcool isopropylique, le 1-butanol, le 2-butanol, la 2-butanone, l'acétonitrile, le tétrachlorure de carbone, le chlorobenzène, le diéthyléther, le diméthyléther, le diméthylformamide (DMF), le diméthylsulfoxyde (DMSO), l'acétate d'éthyle, le chlorométhane, et le dichlorométhane.

**5.** Procédé selon la revendication 1, dans lequel la solution organique de l'agent biologique comprend l'agent biologique et un solvant organique, le solvant organique étant au moins l'un choisi parmi l'acétone, le méthanol, l'éthanol, l'alcool isopropylique, le 1-butanol, le 2-butanol, la 2-butanone, l'acétonitrile, le tétrachlorure de carbone, le chlorobenzène, le diéthyléther, le diméthyléther, le diméthylformamide (DMF), le diméthylsulfoxyde (DMSO), l'acétate d'éthyle, le chlorométhane, et le dichlorométhane.

**6.** Procédé selon la revendication 1, dans lequel au moins l'un parmi le rapport de la quantité prédéterminée d'eau à la quantité de la solution organique du médicament, et le rapport de la quantité prédéterminée d'eau à la solution organique de l'agent biologique, est situé dans la plage allant de 1/100 à 100/1.

**7.** Procédé selon la revendication 1, dans lequel l'au moins un tensioactif dissous est au moins l'un choisi parmi un tensioactif ayant un indice hydro-lipophile (HLB) situé dans la plage allant de 3,5 à 16, le Tween 80, le Tween 60, le Tween 20, l'alcool laurylique éthoxylé, l'alcool tridécylique éthoxylé, un émulsionnant cationique, un émulsionnant anionique, un émulsionnant zwittérionique, et un émulsionnant non-ionique.

**8.** Procédé selon la revendication 1, dans lequel le premier mélange et le deuxième mélange sont homogénéisés séparément par utilisation d'un homogénéisateur à ultrasons pendant un temps situé dans la plage allant de 10 minutes à 200 minutes et à une fréquence située dans la plage allant de 1 Hz à 30 kHz dans un bain d'eau glacée.

**9.** Procédé selon la revendication 1, dans lequel la solution de nano-cristaux du médicament et la solution de nano-particules de l'agent biologique sont soumises ensemble à l'homogénéisation aux ultrasons pendant un temps situé dans la plage allant de 10 minutes à 200 minutes et à une fréquence située dans la plage allant de 1 Hz à 30 kHz dans un bain d'eau glacée.

**10.** Procédé selon la revendication 1, dans lequel le médicament est au moins l'un choisi parmi un agent anti-resténose, un agent antiprolifératif, un agent anti-inflammatoire, un agent antinéoplasique, un agent anticoagulant, un agent anti-fibrine, un agent antithrombotique, un agent antimitotique, un agent antibiotique, un agent antiallergique et un antioxydant, un agent antiprolifératif, un oestrogène, un inhibiteur de protéase, un anticorps, un agent immunodépresseur, un agent cytostatique, un agent cytotoxique, un inhibiteur calcique, un inhibiteur de phosphodiestérase, un inhibiteur de prostaglandine, un complément alimentaire, une vitamine, un agent antiagrégant plaquettaire, et une cellule épithéliale génétiquement modifiée.

**11.** Procédé selon la revendication 1, dans lequel le médicament est au moins l'un choisi parmi le sirolimus, le tacrolimus, le paclitaxel, le clobétasol, la dexaméthasone, la génistéine, l'héparine, le bêta-oestradiol, la rapamycine, l'évérolimus, l'éthylrapamycine, le zotarolimus, ABT-578, le biolimus A9, le docétaxel, le méthotrexate, l'azathioprine, la vincristine, la vinblastine, le fluorouracile, le chlorhydrate de doxorubicine, la mitomycine, la myomycine, le novolimus, l'héparine sodique, une héparine de faible poids moléculaire, un héparinoïde, l'hirudine, l'argatroban, la forskoline, le vapiprost, la prostacycline, un analogue de prostacycline, le dextrane, la D-phe-pro-arg-chlorométhylcétone, le dipyridamole, la glycoprotéine IIb/IIIa, l'hirudine recombinée, la bivalirudine, la nifédipine, la colchicine, la lovastatine, le nitroprusside, la suramine, un inhibiteur de sérotonine, un stéroïde, un inhibiteur de thioprotéase, la triazolopyrimidine, l'oxyde nitrique ou un donneur d'oxyde nitrique, une superoxyde dismutase, un mimétique de superoxyde dismutase, l'oestradiol, l'aspirine, l'angiopeptine, le captopril, le cilazapril, le lisinopril, le permirolast potassique, l'interféron alpha, le RGD bioactif et leurs sels, esters ou analogues.

**12.** Procédé selon la revendication 1, dans lequel le médicament est au moins l'un parmi le sirolimus, le tacrolimus, le paclitaxel et leurs sels, esters ou analogues.

**13.** Procédé selon la revendication 1, dans lequel l'agent biologique est au moins l'un choisi parmi un véhicule de médicament, un excipient, un composant sanguin, un excipient dérivé du sang, un phospholipide, des nanoparticules lipidiques solides, un lipoïde, une vitamine, et une molécule de sucre.

**14.** Procédé selon la revendication 1, dans lequel l'agent biologique est au moins l'un choisi parmi un stéroïde, une vitamine, un oestradiol, un acide gras estérifié, un acide gras non estérifié, le glucose, l'inositol, le L-lactate, une lipoprotéine, un hydrate de carbone, le phosphate tricalcique, le phosphate de calcium précipité, une substance dérivée d'au moins l'un parmi le Phospholipon 80H, le Phospholipon 90H, le Lipoid S75, le Lipoid E80, l'Intralipid 20, le Lipoid EPC, le Lipoid E75, d'humain, d'oeuf et de soja, un lipide obtenu à partir d'oeuf, un lipide obtenu à partir de soja, la phosphatidylcholine, le phosphatidylglycérol, le phosphatidylinositol, la phosphatidylsérine, l'acide phosphatidique, la cardiolipine, et la phosphatidyléthanolamine.

**15.** Procédé selon la revendication 1, dans lequel l'agent biologique est au moins l'un choisi parmi le Lipoid E80 et un phospholipide obtenu à partir d'oeuf.

**16.** Procédé selon la revendication 1, dans lequel les nano-véhicules comprennent le médicament encapsulé dans l'agent biologique, et dans lequel la surface des nano-véhicules est exempte du médicament et les nano-véhicules sont exempts de tout polymère.

**17.** Procédé selon la revendication 1, dans lequel la taille des nano-véhicules est située dans la plage allant de 10 nm à 5000 nm.

**FIG. 1**

**FIG. 2**

**FIG. 3**

**FIG. 4**

FIG. 5

**FIG. 6**

**FIG. 7**

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004069169 A2


### Non-patent literature cited in the description

- Particle Size Distribution Measurement for Millimeters to Nanometers and from Rods to Platelets. **BOWEN P.** Journal of Dispersion Science and Technology. Taylor and Francis Group, 01 January 2002, vol. 23, 631-662